# EUROPEAN PATENT APPLICATION

(11) **EP 1 985 242 A1**
(43) Date of publication of application: **29.10.2008**
(21) Application number: 08155043.6
(22) Date of filing: 23.04.2008
(51) Int. Cl.: A61B 17/064, A61B 17/00, A61B 19/00, A61B 17/04

(54) **Tissue staple**

(30) Priority: 23.04.2007 US 738605
(71) Applicant: Medtronic Vascular, Inc., Santa Rosa CA 95403 (US)
(72) Inventor: Sater, Ghaleb, Acton, MA 01720 (US); Spurchise, Matthew, Peabody, MA 01960 (US); Mas, Juan-Pablo, Somerville, MA 02144 (US)
(74) Representative: Jilderda, Anne Ayolt

(57) **Abstract**

A stapling arrangement and method for closing a percutaneously formed arteriotomy includes a staple with a plurality of staple prongs (12A-12D) commonly connected at their proximal ends and having distal ends adapted to engage tissue on opposite sides of the arteriotomy. The prongs have for instance an arrangement of stops (26) configured to present different degrees of resistance to advancement through tissue.

## Description

The invention relates to a tissue staple comprising a plurality of prongs having proximal and distal ends, the prongs, having sharp distal tips, being commonly connected at their proximal ends and presenting at least one first prong and at least one second prong, said at least one second prong being disposed generally opposite said at least one first prong. The invention relates, particularly, to a staple for closing an arteriotomy.

A staple of the kind described in the opening paragraph may be used in systems for closing a percutaneous puncture in a blood vessel during a vascular procedure. Various cardiovascular procedures, such as angioplasty, stent placement, atherectomy, among others, are performed by inserting into and manipulating within the vasculature, wires and catheters adapted to perform those procedures. Access to the vasculature typically is through the femoral artery and is percutaneous, involving insertion of a needle in the region of the groin to form a track and to puncture and create an arteriotomy in the femoral artery. A guidewire then is advanced through the needle and into the femoral artery. The needle then is removed. An introducer sheath is then advanced over the guidewire. The wire and sheath provide access into the femoral artery, through the arteriotomy, for catheters or other instrumentalities in order to perform the selected procedure.

After the procedure has been completed, the procedural devices are removed and the arteriotomy must be closed. A number of techniques are known to facilitate closure and healing of the arteriotomy. These include application of pressure at the puncture site for a relatively extended length of time or the use of biological adhesives or plugs adapted to seal the arteriotomy, among others. Also among the techniques for closing the arteriotomy is the use of a staple system such as described in U.S. patents 6,506,210, 6,767,356 and 7,074,232 to Kanner et al., of which the disclosures of the devices and methods are hereby incorporated by reference. The Kanner patents describe a system by which the original introducer sheath is removed, leaving the guidewire in place. Then, an assembly that includes a sheath and dilator is advanced along the indwelling guidewire to bring the distal end of the sheath into proximity to the arteriotomy. The sheath also carries an arrangement of wire-like stabilizers that, together with the dilator, pass through the arteriotomy into the artery. The system enables the portions of the stabilizer wires disposed within the artery to be formed into a temporarily enlarged shape that prevents removal of the wires through the arteriotomy. The stabilizers and distal end of the sheath are drawn together to grip the tissue about the arteriotomy and thereby secure and fix the position of the distal end of the sheath over and in alignment with the arteriotomy. The dilator and guidewire then can be removed through the sheath, leaving the sheath in place adjacent the outer surface of the artery with the stabilizers within the artery, holding the sheath in place in readiness to provide direct access to the arteriotomy.

A catheter-like stapling device, with a staple carried in its distal end, then is advanced through the sheath to locate the staple in proximity to the arteriotomy. As described more fully in the Kanner patents, the stapler and sheath include mechanisms by which the staple, when advanced through the sheath, will be oriented in registry with and at a fixed distance from the arteriotomy. When the stapler is actuated, the prongs of the staple will expand and advance toward and into the arterial wall on opposite sides of the arteriotomy. Continued operation of the stapling mechanism draws the prongs of the staple together to draw the edges of the arteriotomy together into approximation, then releases the staple. The stabilizers return to a linear shape enabling their withdrawal. With the staple deployed and having closed the arteriotomy, the stapling mechanism and sheath may be removed, leaving the staple in place.

During the cardiovascular procedure, the angle of approach of the guidewires, catheters and other devices relative to the femoral artery may be selected by the physician to accommodate the particular anatomy and device in use. When closing the arteriotomy with a staple, however, it is desirable that the staple approach and engage the artery at an angle such that all of the sharp tips of the prongs of the staple may engage the tissue and penetrate to approximately the same depth. Although, ideally, such an angle would be close to orthogonal, in practice, the system is positioned so that the stapling and delivery system is oriented at an approach angle of about 45 degrees.

It is desirable that the prongs of the staple pierce the arterial wall to a depth sufficiently that when the prongs are closed, they will draw the edges of the arteriotomy together to close the arteriotomy, while avoiding projection into the arterial lumen. The prongs do not necessarily have to go very deep into the arterial wall. It may be sufficient if they engage only partially the vessel wall while also engaging surrounding connective tissue, such as the femoral sheath. In order to control the extent to which the prongs may be advanced through the tissue to avoid penetrating into the lumen of the artery, each of the prongs described in the Kanner patents may be provided with a tissue stop adapted to engage tissue to present increased resistance to penetration of the prongs beyond a specified limit.

By approaching the tissue at an angle of about 45 degrees, one or more of the prongs of the staple will reach a tissue depth before the other prongs reach that depth, the former being referred to in this description as "leading" and the latter as "trailing". Consequently, when the staple is crimped to close the prongs, the leading and trailing prongs may engage the vascular and surrounding tissue differently than if the staple engaged the vessel orthogonally. Although that might not adversely effect closing of the arteriotomy, it would be desirable to have a more uniform prong-tissue engagement.

The present invention relates to modified staples including modified embodiments of the staple described in the Kanner patents by which the tissue-prong engagement and penetration depth of the leading and trailing staples may be more uniform and in which the security of engagement of the staple prongs with the artery wall may be enhanced.

In order to attain the above described object a tissue staple of the kind described in the opening paragraph is characterized in that said staple prongs are configured to present different degrees of resistance to advancement through tissue such that said at least one first prong presents a greater degree of resistance to advancement through tissue than said at least one second prong. Accordingly, the staples are modified so that prongs are configured to present different degrees of resistance to advancement through tissue. This may be achieved by varying the placement, dimensions, or configuration of tissue stops on the prongs. Alternatively the dimensions, particularly the cross-sectional dimensions, of the staple prongs may be varied among the staple prongs or the prongs may be given varying surface properties in order to increase or decrease said penetration resistance, for instance by coating or treating selected surfaces.

In one aspect of the invention the staple is configured so that the leading prongs present greater resistance to advancement through tissue than the trailing prongs. This enables the prongs, each of which is capable of being moved independently of the others, to cooperate with the tissue such that the trailing prongs can be caused to penetrate the tissue to a depth that is closer to the penetration depth of the leading prongs. In accordance with one aspect of the invention, the staple may be configured so that the leading prongs will present greater resistance to advancement through tissue than the trailing prongs. That results in a dynamic interaction with tissue by which the penetration of the leading and trailing prongs will be more nearly equal than if all of the prongs presented the same resistance to advancement through tissue. Other aspects of the invention include the number and placement of tissue engaging surfaces of the staples. The invention also relates to a method of stapling an arteriotomy.

The invention will be described in greater detail with reference to a number of illustrative embodiments and the accompanying drawings, in which
- FIGS. 1-3: are isometric views of a prior art staple in formed, opened and deployed positions, respectively;
- FIG. 4: is a diagrammatic plan view of an artery with an arteriotomy in which a portion of the stabilizers of a delivery system is shown in engagement with the arteriotomy and vessel lumen;
- FIG. 4A: is a diagrammatic illustration of an arteriotomy with edges approximated by the delivery system and showing the position at which the staple prongs pierce the tissue on opposite sides of the arteriotomy;
- FIG. 5: is a diagrammatic sectional illustration of the stabilization system shown partially in FIG. 4, as seen along the section line 5-5 of FIG. 4, including the sheath and deployed stabilizers;
- FIG. 6: is an illustration of a modified staple in accordance with the present invention;
- FIG. 6A: is a diagrammatic illustration of one manner in which the staple of FIG. 6 may dynamically engage the tissue about the arteriotomy;
- FIG. 6B: is a diagrammatic illustration of another manner in which the staple of FIG. 6 may dynamically engage the tissue about the arteriotomy;
- FIG. 7: is an illustration of another modified embodiment of the invention in which the leading prongs are provided with full stops and the trailing prongs are provided with lateral wings to increase the tissue gathering area without significantly increasing the resistance of the trailing prongs to penetrate through tissue;
- FIG. 8: is an illustration of a further modified embodiment of the invention in which leading and trailing prongs are provided with stops that are located at different positions along the lengths of the prongs; and
- FIG. 9: is an illustration of a two-prong staple with a stop on only one prong.

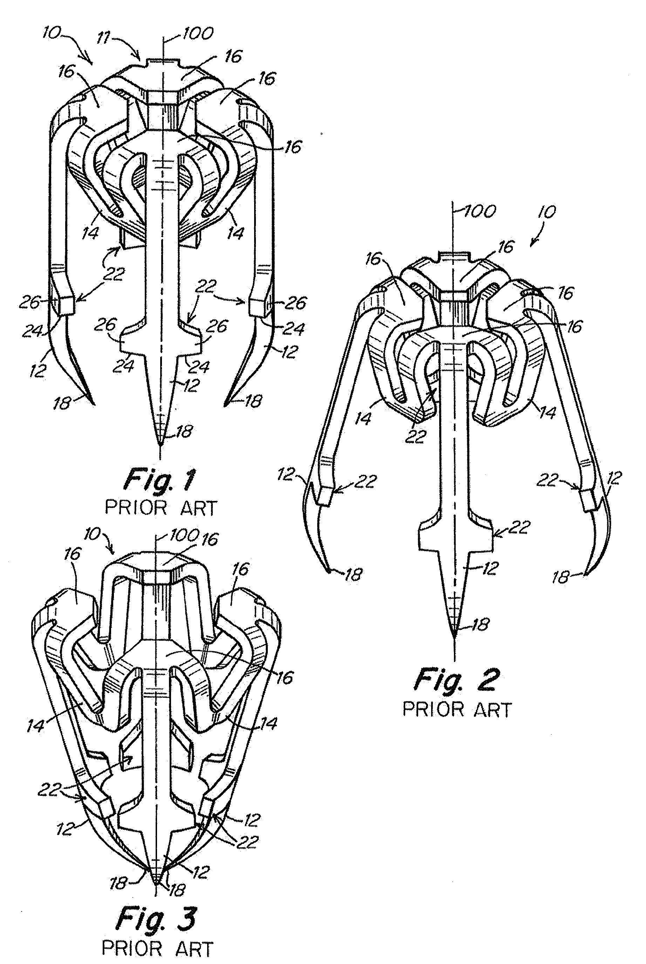

FIG. 1 illustrates a staple as described in the Kanner patents. The staple has a central axis 100 and may be formed to include a plurality of prongs 12 that are commonly connected at a proximal crown 11. By way of dimensional example, the staple may be of the order of five to six millimeters long and may have a diameter of approximately three millimeters. The prongs 12 extend in a distal direction, parallel to the axis 100, where they terminate in sharpened distal tips 18. As described in further detail in the Kanner patents, the crown 11 may be defined by a series of tabs 14 and shoulders 16 that cooperate with a delivery device in a manner that enables the prongs 12 first to be spread apart (FIG. 2) and then, as the staple is advanced distally toward and into tissue, to cause the prongs to close (FIG. 3). The arrangement of the crown 11 with its tabs 14 and shoulders 16 by which adjacent prongs are connected to each other, provides an ability of the prongs to be moved independently relative to each other. For example, if one prong meets a greater degree of resistance than another, as from tissue variations or other causes, that does not prevent other prongs meeting less or different resistance from moving relatively independently.

The prongs may be provided with tissue stops 22 defined by distally facing surfaces 24 formed on lateral wing-like projections 26 on each of the prongs. The tissue stops present increased resistance to advancement of the prongs as they are urged through tissue to control the penetration depth of the prongs and avoid damage to the innermost, intimal, layer of the artery. By way of example, the tissue stops may be located less than one millimeter from the distal tip of its associated prong, for example, about 0.8 millimeters.

The wings 26 on which the tissue stops 24 are defined also extend transversely so that their inwardly facing (toward the axis) surfaces 28 will present an increased area to gather tissue as the prongs are closed. When the prongs are engaged with tissue and the staple is closed, the inwardly facing surfaces of the wings will gather tissue on opposite sides of the arteriotomy and draw or pucker that tissue together. It should be noted that it is not essential that the inwardly facing surfaces 28 of the wings be embedded in the arterial wall in order to facilitate closure of the arteriotomy. The immediate region about the artery includes connective tissue and, in the case of the femoral artery, the femoral sheath, so that engagement of the wings 26 with those tissues will also draw together the edges of the arteriotomy.

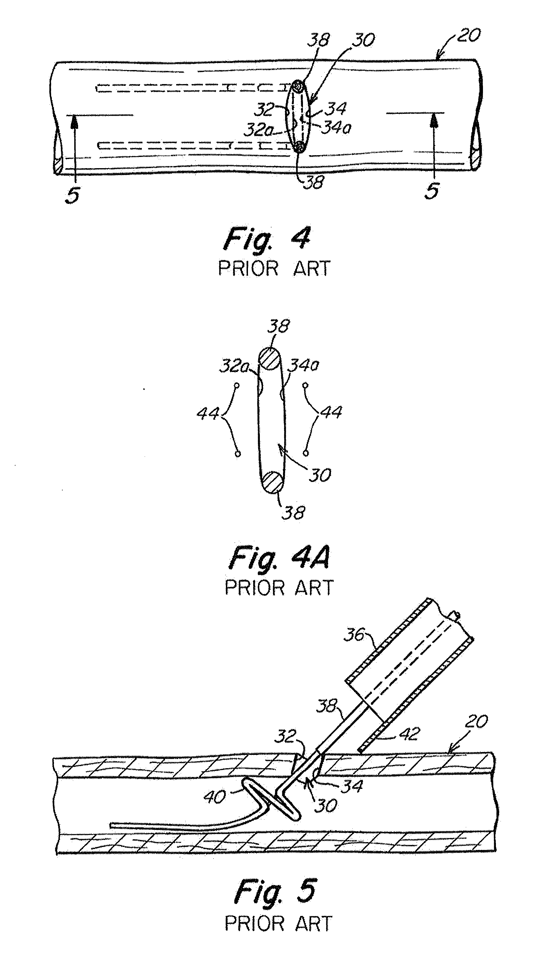

FIGS. 4 and 5 illustrate, diagrammatically, an artery 20 that has been punctured to form an arteriotomy 30. Arterial anatomy is such that when a puncture is made in the wall of an artery, the puncture assumes the form of a circumferentially oriented slit defined by arteriotomy edges 32, 34. This circumferential orientation results from the cellular structure of an artery, particularly its medial layer, by which it accommodates, by expansion and contraction, variation in hemostatic pressure.

As described in further detail in the above-mentioned Kanner patents, the closure system includes an arrangement of a sheath 36 and wire-like stabilizers 38 that extend through and engage the ends of the arteriotomy to position and align the sheath 36 with the arteriotomy 30 and to stabilize it in that position for subsequent advancement of the stapler. The stabilizers 38 may be mounted to the sheath 36 and may be operated to change configuration temporarily from a linear configuration to a non-linear configuration, such as the zigzag arrangement 40 shown in FIG. 5. The stabilizers are mounted with respect to the sheath 36 so that they engage the lateral ends of the arteriotomy 30 and may be spaced to stretch the arteriotomy 30 laterally to tension the edges 32, 34 so that the edges 32, 34 will be drawn toward each other as suggested in phantom at 32a, 34a in FIG. 4. The non-linear configuration 40 of the stabilizers 38 serves to retain the stabilizers in place and prevents them from being withdrawn through the arteriotomy. Additionally, the sheath 36 may be provided with a gauge 42 that projects a fixed distance distally beyond the distal end of the sheath to engage the outer surface of the vessel or surrounding tissue when the distal end of the sheath has reached that distance from the outer surface of the vessel 20. Thus, the foregoing arrangement serves to position the sheath 36 in proper alignment with the arteriotomy and the stabilizers 38 serve to approximate the edges 32, 3 of the arteriotomy in readiness for stapling. FIG. 4A illustrates, diagrammatically, the locations 44 at which the prongs of the staple may engage the tissue opposite sides of the arteriotomy.

In accordance with the invention, the arteriotomy staple may be modified so that fewer than all of the prongs include tissue stops 22 or wings 26 with the result that some prongs will present more or less resistance than others to advancement through tissue. By providing prongs with varying degrees of resistance to advancement, the dynamic interaction between the tissue about the arteriotomy and the staple may be affected in a manner that compensates somewhat for the non-orthogonal approach angle of the staple with respect to the artery. Staples thus may be provided with modified arrangements of wings 26 and tissue stops 22 to facilitate penetration of all of the prongs to a sufficient depth and degree of engagement with tissue that may result in enhanced arteriotomy closure.

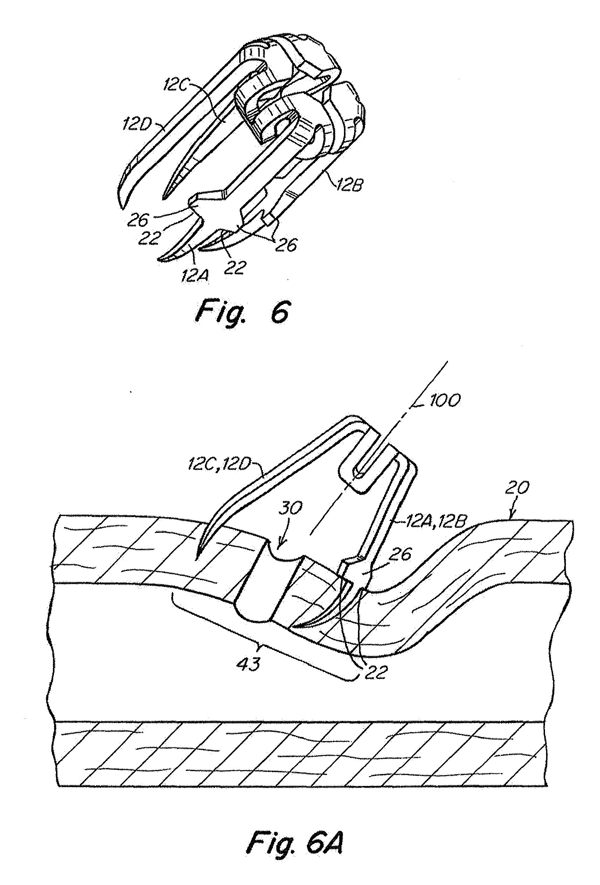

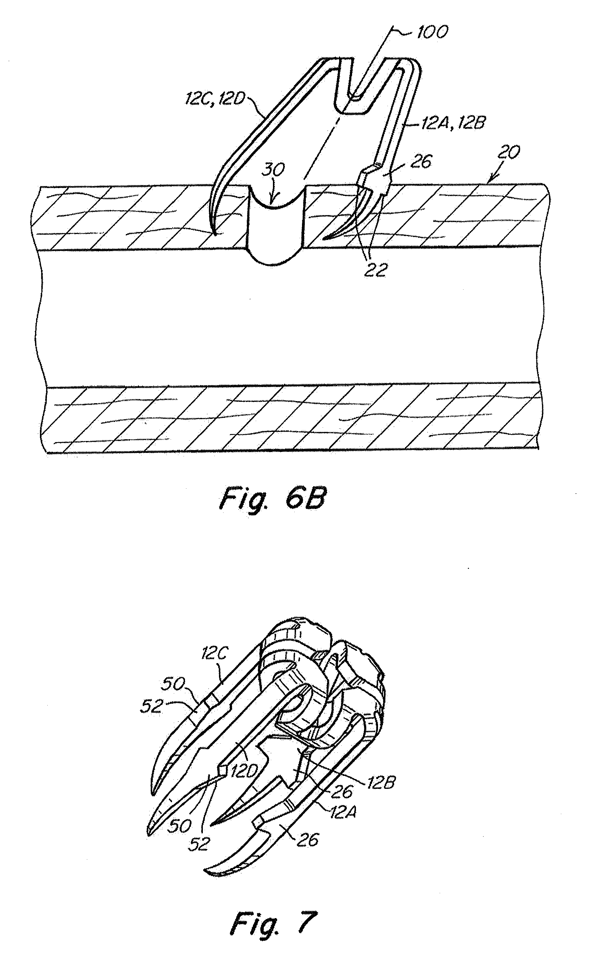

FIG. 6 illustrates one embodiment of a modified staple in which some, but fewer than all, of the prongs are provided with tissue stops 22 and wings 26 and in which the angle of approach to the vessel is about 45 degrees. For convenience of description prongs that will be first to contact the vessel wall may be referred to as "leading" the other, "trailing," prongs. In this embodiment, the leading prongs 12A, 12B are provided with tissue stops 22 and wings 26 while the trailing prongs 12C, 12D have no stops 22 or wings 26. With this arrangement, and with an approach angle of the order of 45 degrees, the tissue stops 22 of the leading prongs will, at some point in their advancement through tissue, be presented with significant resistance to further penetration of the leading prongs. As the force of advancement is maintained, the varying resistance presented to the different prongs is believed to affect the dynamic interaction between the tissue and the staple, either by causing distortion of the staple, or of the tissue, or a combination of both. In the illustrative embodiments, the leading prongs are adapted to engage tissue proximally of the arteriotomy and the trailing prongs are adapted to engage the distal side of the arteriotomy.

Under one theory of the dynamic action of the asymmetrical staple of FIG. 6, as the force of advancement is maintained, the tissue engaged by the stops 22 and wings 26 may be pressed distally, causing deformation of part of the vessel wall by which the tissue about the arteriotomy may be distorted and reoriented to be presented more orthogonally to the direction of advancement of the staple. Consequently, the tissue about the arteriotomy may be oriented briefly to the configuration suggested diagrammatically in FIG. 6A in which the stops and wings on the leading prongs 12A, 12B have forced a segment 43 of the arterial wall to distort so that the vessel surface about the arteriotomy is distorted to be closer to orthogonal to the axis 100 of the staple. Consequently, the trailing prongs 12C and 12D are in less of a trailing position relative to the region of the arteriotomy region with respect to the leading prongs 12A, 12B. The absence of tissue stops on the trailing prongs 12C, 12D enables those prongs to continue to penetrate without affecting the shape of the tissue about the arteriotomy or resisting the brief shape change caused by the leading stops. Consequently, during the final phase of the stapling when the prongs are brought together to draw the tissue about the arteriotomy together the prongs on both sides of the arteriotomy will be in firm engagement with the tissue and will close the arteriotomy.

In another theory of operation, the trailing prongs, presenting less resistance to advancement through tissue, may continue to move distally relative to the leading prongs that will have met increased tissue resistance. The manner in which the prongs are connected with the crown configuration of tabs 14 and shoulders 16 provides some freedom of movement of the prongs independently of the other prongs. Thus, the crown of the staple may deform under the influence of the driving force such that the trailing prongs may continue to advance as suggested in FIG. 6B. As can be seen in FIG. 6B, the crown and its components have been deformed to permit the trailing prongs to advance further than the leading prongs. Consequently, the trailing prongs can advance through tissue along an axial direction to a depth greater than would be achieved otherwise.

The dynamic interaction between the staple and the tissue into which it is driven may depend upon a number of patient-related factors, particularly the condition of the artery and the surrounding tissue (e.g., wall thickness, existence or degree of stenosis, calcification of vessel, scar tissue, thickness of fascia and connective tissue). In some instances, the physician may find it desirable to employ a staple (or to reorient the position of the staple) so that the trailing prongs present greater resistance to penetration than the leading prongs. In such an arrangement, for example, the staple shown in FIG. 6 would be presented in a reoriented position in which the stop surfaces 22 and wings 26 are disposed on the trailing prongs 12C, 12D. Such a staple might be desirable where the anatomy of the artery and surrounding tissue is such that the trailing prongs would be expected to meet significant resistance to tissue penetration such that the advancement of the staple would stop before the distal tips of the leading prongs reached the innermost luminal surface of the vessel.

FIG. 7 illustrates a further embodiment of a staple with the further modification that the trailing prongs 12C, 12D are provided with reduced width, transversely extending wings 50 having a wedge-like stop surface 52 that presents only partial resistance to advancement of the prong through tissue. The principal function of these wings is to provide an increased area along its inwardly facing surface sufficient to enhance the drawing in and gathering of tissue when the staple is closed. The dimensions and angles associated with the partial stop surfaces may be varied to provide the desired balance between resistance to axial advancement and inwardly facing wing area.

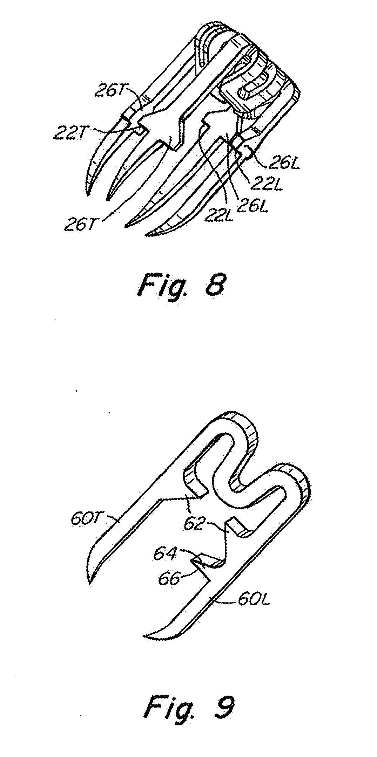

FIG. 8 illustrates a further embodiment of the invention in which the leading prongs and the trailing prongs are provided with stops 22L and wings 26L that present substantially equal resistance to tissue penetration but in which the stops 22T and wings 26T on the trailing prongs are disposed more distally than the stops 22L on the leading prongs. The stops and wings are located to be approximately level when the staple is oriented at the approach angle. With this arrangement, and depending on the characteristics of the tissue and anatomy into which the staple will be advanced, the stops 22L, 22T on all of the prongs may engage tissue at or about the same level in the tissue.

FIG. 9 illustrates another embodiment of the invention applied to a two-prong staple. As shown in FIG. 9, the staple has a leading prong 60L and a trailing prong 60T. Each of the prongs includes a wedge-like ramp 62 that is designed to cooperate with a corresponding stapling device to first open, then advance, then close the staple. The leading prong 60L also includes an inward projection 64 that defines a stop surface 66 adapted to engage tissue to present greater resistance to advancement through tissue than the other, trailing prong.

It should be understood that the foregoing description of the several dynamic interactions between the staple and the tissue about the arteriotomy are not intended to be exclusive and that other dynamic effects may occur with the use of the staple of the present invention. It also should be understood that the foregoing description of the invention is intended merely to be illustrative and that other embodiments, modifications and equivalents may be apparent to those skilled in the art while remaining within the scope of the invention.

## Claims

1. A tissue staple comprising a plurality of prongs having proximal and distal ends, the prongs, having sharp distal tips, being commonly connected at their proximal ends and presenting at least one first prong and at least one second prong, said at least one second prong being disposed generally opposite said at least one first prong, **characterized in that** said prongs are configured to present different degrees of resistance to advancement through tissue such that said at least one first prong presents a greater degree of resistance to advancement through tissue than said at least one second prong.

2. A tissue staple according to claim 1 **characterized in that** at least one, but fewer than all, of the prongs has a tissue stop disposed along the prong, the tissue stop defining a surface at least partly facing in a distal direction to provide greater resistance to advancement of the prong through tissue, than a prong without a tissue stop.

3. A tissue staple according to claim 1 or 2 **characterized in that** at least one first prong has a tissue stop disposed proximally of its distal end, the tissue stop defining a surface at least partly facing in a distal direction to present additional resistance to advancement of the prong through tissue.

4. A tissue staple according to claim 2 or 3 **characterized in that** said tissue stop is formed on a wing that extends transversely of the length of its associated prong, the wing providing an enlarged inwardly facing area adapted to draw tissue inwardly during inward closure of the staple prongs.

5. A tissue staple according to any of the preceding claims **characterized in that** each first prong has a tissue stop disposed proximally of its distal end, the tissue stop defining a surface facing in a distal direction to present additional resistance to advancement of the prong through tissue; and **in that** each second prong has laterally extending wings that have a surface facing partly in a distal direction, the degree of resistance to advancement through tissue of each second prong being substantially less than that of each first prong.

6. A tissue staple according to claim 1 **characterized in that** said prongs comprise a single first prong and a single second prong, **in that** said single first prong has a surface that faces at least partly in a distal direction to present additional resistance to advancement of that prong through tissue, and **in that** said second prong is configured to present substantial less resistance to advancement through tissue than said first prong.

7. A tissue staple according to any of the preceding claims **characterized in that** said common connection at the proximal ends of the prongs is constructed and arranged to enable independent movement of the prongs relative to each other.

8. A tissue staple according to claim 7 wherein the common connection enables the prongs to shift axially relative to each other.

9. A staple according to any of the preceding claims **characterized in that** said at least one first prong comprise at least one leading prong and said at least one second prong comprise at least one trailing prong.

10. A tissue staple according to claim 9 **characterized in that** at least one leading prong has a tissue stop and at least one trailing prong is free of tissue stops, such that an advancement of said at least one trailing prong through tissue may continue independently after the advancement of said at least one leading prong has been materially resisted by the tissue.

11. A tissue staple according to claim 9 **characterized in that** each leading prong has a tissue stop and each trailing prong is free of tissue stops, such that an advancement of each leading prong against tissue may cause the tissue to deform and such that each trailing prong may pass through tissue without presenting substantial resistance to the ability of the tissue to deform.
